# EUROPEAN PATENT APPLICATION

(11) **EP 4 368 117 A1**
(43) Date of publication of application: **15.05.2024**
(21) Application number: 23206175.4
(22) Date of filing: 26.10.2023
(51) Int. Cl.: A61B 8/08, A61B 8/00

(54) **ULTRASOUND DIAGNOSTIC APPARATUS AND CONTROL METHOD OF ULTRASOUND DIAGNOSTIC APPARATUS**

(30) Priority: 27.10.2022 JP 2022172588
(71) Applicant: FUJIFILM Corporation, Tokyo 106-8620 (JP)
(72) Inventor: OKADA, Yuto, Kanagawa, 258-8538 (JP)
(74) Representative: Hughes, Andrea Michelle

(57) **Abstract**

Provided are a control method of an ultrasound diagnostic apparatus and an ultrasound diagnostic apparatus that enable a user to easily confirm a detection result without displaying a detailed detection result of a stool on a screen.

An ultrasound diagnostic apparatus includes: an ultrasound probe (1); an image acquisition unit (31) that acquires an ultrasound image of a subject using the ultrasound probe; a monitor (23) that displays the ultrasound image; a stool detection unit (24) that detects a stool from the ultrasound image; a selection button (25) that is displayed on the monitor and is used for a user to select whether or not to display a detection result by the stool detection unit; and a display controller (22) that displays the selection button on the monitor in a display mode corresponding to the detection result by the stool detection unit.

## Description

### BACKGROUND OF THE INVENTION

### 1. Field of the Invention

The present invention relates to an ultrasound diagnostic apparatus and a control method of an ultrasound diagnostic apparatus for detecting a specific target from an ultrasound image.

### 2. Description of the Related Art

Conventionally, a so-called ultrasound diagnostic apparatus has been used to examine a stool present inside a subject. In this case, a user of the ultrasound diagnostic apparatus, such as a doctor, often determines the presence or absence of the stool inside the subject, the properties of the stool, and the like by confirming an ultrasound image. However, usually, the user requires a certain proficiency level or higher to confirm the ultrasound image to determine the presence or absence of the stool and the properties of the stool. In that respect, a technology has been developed to automatically perform the detection of the stool inside the subject and the evaluation of the properties of the detected stool, for example, as disclosed in JP2016-195748A.

### SUMMARY OF THE INVENTION

Meanwhile, in the technology disclosed in JP2016-195748A, since the detailed detection result of the stool is always displayed, the display of the detailed detection result may sometimes hinder the examination for some users.

The present invention has been made in order to solve such a conventional problem, and an object of the present invention is to provide an ultrasound diagnostic apparatus and a control method of an ultrasound diagnostic apparatus that enable a user to easily confirm a detection result without displaying a detailed detection result of a stool on a screen.

According to the following configuration, the above-described object can be achieved.
[1] An ultrasound diagnostic apparatus comprising:
   an ultrasound probe;
   an image acquisition unit configured to acquire an ultrasound image of a subject using the ultrasound probe;
   a monitor configured to display the ultrasound image;
   a stool detection unit configured to detect a stool from the ultrasound image;
   a selection button displayed on the monitor and used for a user to select whether or not to display a detection result by the stool detection unit; and
   a display controller configured to display the selection button on the monitor in a display mode corresponding to the detection result by the stool detection unit.
[2] The ultrasound diagnostic apparatus according to [1], further comprising:
   a stool property determination unit configured to determine a property of the stool detected by the stool detection unit,
   in which the display controller is configured to change the display mode of the selection button according to the property of the stool determined by the stool property determination unit.
[3] The ultrasound diagnostic apparatus according to [1] or [2],
   in which the stool detection unit is configured to detect presence or absence of the stool in a deep-side region of a high-brightness part on the ultrasound image, and
   the display controller is configured to change the display mode of the selection button according to the presence or absence of the stool in the deep-side region detected by the stool detection unit.
[4] The ultrasound diagnostic apparatus according to [3],
   in which the display controller is configured to change a change range of the display mode on the selection button according to a presence range of the stool in the deep-side region detected by the stool detection unit.
[5] The ultrasound diagnostic apparatus according to any one of [1] to [4],
   in which the display controller is configured to display a presence range of the stool detected by the stool detection unit on the monitor with a surrounding frame line.
[6] The ultrasound diagnostic apparatus according to any one of [1] to [5],
   in which the display controller is configured to display on the monitor that no stool is detected in a case where the stool is not detected by the stool detection unit.
[7] A control method of an ultrasound diagnostic apparatus including an ultrasound probe, an image acquisition unit, a monitor, a stool detection unit, a selection button, and a display controller, the control method comprising:
   causing the image acquisition unit to acquire an ultrasound image of a subject using the ultrasound probe;
   causing the monitor to display the ultrasound image;
   causing the stool detection unit to detect a stool from the ultrasound image; and
   causing the display controller to display the selection button used for a user to select whether or not to display a detection result of stool detection, on the monitor in a display mode corresponding to the detection result of the stool detection.

According to the present invention, an ultrasound diagnostic apparatus comprises: an ultrasound probe; an image acquisition unit that acquires an ultrasound image of a subject using the ultrasound probe; a monitor that displays the ultrasound image; a stool detection unit that detects a stool from the ultrasound image; a selection button that is displayed on the monitor and is used for a user to select whether or not to display a detection result by the stool detection unit; and a display controller that displays the selection button on the monitor in a display mode corresponding to the detection result by the stool detection unit. Therefore, the user can easily confirm the detection result without displaying the detailed detection result of the stool on the screen.

### BRIEF DESCRIPTION OF THE DRAWINGS

Fig. 1 is a block diagram showing a configuration of an ultrasound diagnostic apparatus according to Embodiment 1 of the present invention.
Fig. 2 is a block diagram showing a configuration of a transmission and reception circuit in Embodiment 1 of the present invention.
Fig. 3 is a block diagram showing a configuration of an image generation unit in Embodiment 1 of the present invention.
Fig. 4 is a diagram showing an example of a selection button in a case where detection processing is not operating in Embodiment 1 of the present invention.
Fig. 5 is a diagram showing an example of the selection button in a case where no stool is detected in Embodiment 1 of the present invention.
Fig. 6 is a diagram showing an example of the selection button in a case where a stool is detected in Embodiment 1 of the present invention.
Fig. 7 is a flowchart showing an operation of the ultrasound diagnostic apparatus according to Embodiment 1 of the present invention.
Fig. 8 is a diagram showing an example of the selection button in a case where the stool is detected in a modification example of Embodiment 1 of the present invention.
Fig. 9 is a diagram showing an example of the selection button in a case where the stool is detected in another modification example of Embodiment 1 of the present invention.
Fig. 10 is a block diagram showing a configuration of an ultrasound diagnostic apparatus according to Embodiment 2 of the present invention.

### DESCRIPTION OF THE PREFERRED EMBODIMENTS

Hereinafter, embodiments of the present invention will be described with reference to the accompanying drawings.

The description of configuration requirements to be described below is made based on a representative embodiment of the present invention, but the present invention is not limited to such an embodiment.

In the present specification, a numerical range represented by "to" means a range including numerical values described before and after "to" as a lower limit value and an upper limit value, respectively.

In the present specification, "same" and "identical" include an error range generally allowed in the technical field.

### Embodiment 1

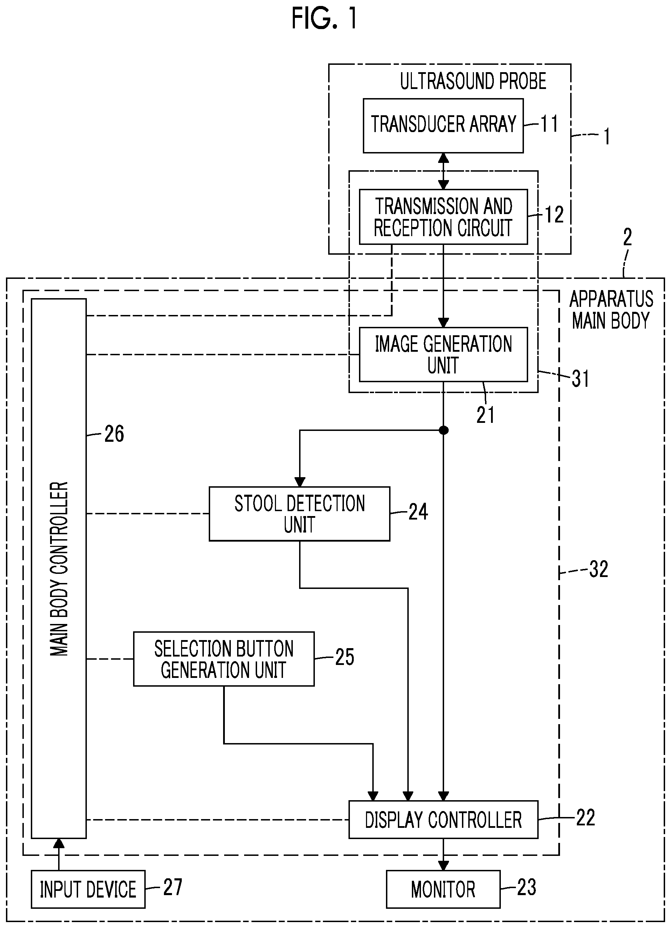

Fig. 1 shows a configuration of an ultrasound diagnostic apparatus according to an embodiment of the present invention. The ultrasound diagnostic apparatus comprises an ultrasound probe 1 and an apparatus main body 2 connected to the ultrasound probe 1. The ultrasound diagnostic apparatus of Embodiment 1 of the present invention is used to examine a stool present inside a subject.

The ultrasound probe 1 includes a transducer array 11. A transmission and reception circuit 12 is connected to the transducer array 11.

The apparatus main body 2 includes an image generation unit 21 connected to the transmission and reception circuit 12 of the ultrasound probe 1. A display controller 22 and a monitor 23 are sequentially connected to the image generation unit 21. In addition, a stool detection unit 24 is connected to the image generation unit 21. The stool detection unit 24 is connected to the display controller 22. In addition, the apparatus main body 2 includes a selection button generation unit 25. The selection button generation unit 25 is connected to the display controller 22. In addition, a main body controller 26 is connected to the transmission and reception circuit 12, the image generation unit 21, the display controller 22, the stool detection unit 24, and the selection button generation unit 25. An input device 27 is connected to the main body controller 26. In addition, the image generation unit 21, the display controller 22, the stool detection unit 24, the selection button generation unit 25, and the main body controller 26 constitute a processor 32 for the apparatus main body 2.

The transducer array 11 of the ultrasound probe 1 includes a plurality of ultrasound transducers one-dimensionally or two-dimensionally arranged. Each of these ultrasound transducers transmits an ultrasound wave in accordance with a drive signal supplied from the transmission and reception circuit 12 and receives an ultrasound echo from a subject to output a signal based on the ultrasound echo. For example, each ultrasound transducer is composed of a piezoelectric body consisting of a piezoelectric ceramic represented by lead zirconate titanate (PZT), a polymer piezoelectric element represented by poly vinylidene di fluoride (PVDF), a piezoelectric single crystal represented by lead magnesium niobate-lead titanate (PMN-PT), or the like, and electrodes formed at both ends of the piezoelectric body.

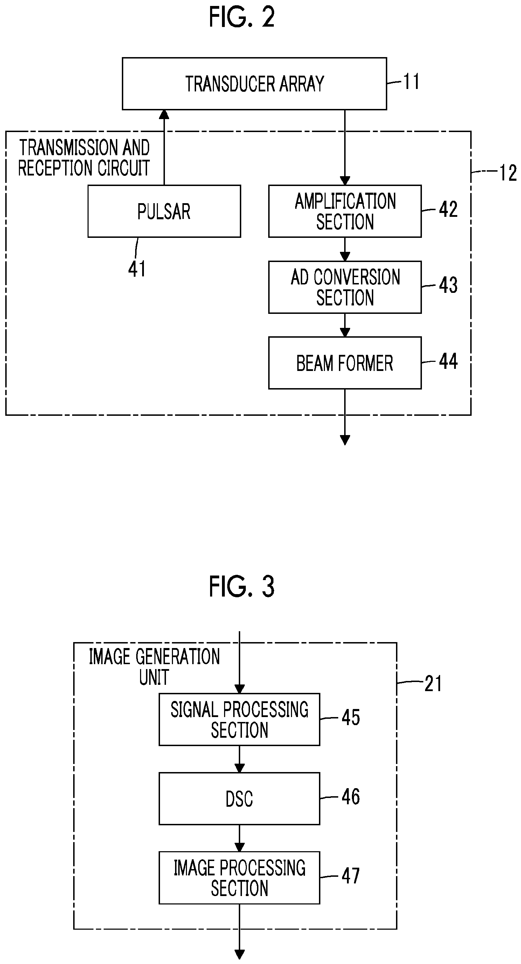

The transmission and reception circuit 12 transmits the ultrasound wave from the transducer array 11 and generates a sound ray signal based on a reception signal acquired by the transducer array 11, under the control of the main body controller 26. As shown in Fig. 2, the transmission and reception circuit 12 includes a pulsar 41 connected to the transducer array 11, and an amplification section 42, an analog-to-digital (AD) conversion section 43, and a beam former 44 that are sequentially connected in series to the transducer array 11.

The pulsar 41 includes, for example, a plurality of pulse generators, and adjusts an amount of delay of each of drive signals and supplies the drive signals to the plurality of ultrasound transducers such that ultrasound waves transmitted from the plurality of ultrasound transducers of the transducer array 11 form an ultrasound beam based on a transmission delay pattern selected according to a control signal from the main body controller 26. In this way, in a case where a pulsed or continuous wave-like voltage is applied to the electrodes of the ultrasound transducer of the transducer array 11, the piezoelectric body expands and contracts to generate a pulsed or continuous wave-like ultrasound wave from each of the ultrasound transducers, whereby an ultrasound beam is formed from the combined wave of these ultrasound waves.

The transmitted ultrasound beam is reflected in, for example, a target such as a site of the subject and propagates toward the transducer array 11 of the ultrasound probe 1. The ultrasound echo propagating toward the transducer array 11 in this way is received by each of the ultrasound transducers constituting the transducer array 11. In this case, each of the ultrasound transducers constituting the transducer array 11 receives the propagating ultrasound echo to expand and contract to generate a reception signal, which is an electrical signal, and outputs these reception signals to the amplification section 42.

The amplification section 42 amplifies the signal input from each of the ultrasound transducers constituting the transducer array 11 and transmits the amplified signal to the AD conversion section 43. The AD conversion section 43 converts the signal transmitted from the amplification section 42 into digital reception data. The beam former 44 performs so-called reception focus processing by applying and adding a delay to each reception data received from the AD conversion section 43. By this reception focus processing, each reception data converted by the AD conversion section 43 is phase-added, and a sound ray signal in which the focus of the ultrasound echo is narrowed down is acquired.

As shown in Fig. 3, the image generation unit 21 has a configuration in which a signal processing section 45, a digital scan converter (DSC) 46, and an image processing section 47 are sequentially connected in series.

The signal processing section 45 generates a B-mode image signal, which is tomographic image information regarding tissues inside the subject, by performing, on the sound ray signal received from the transmission and reception circuit 12, correction of the attenuation due to the distance according to the depth of the reflection position of the ultrasound wave using a sound velocity value set by the main body controller 26 and then performing envelope detection processing.

The DSC 46 converts (raster-converts) the B-mode image signal generated by the signal processing section 45 into an image signal in accordance with a normal television signal scanning method.

The image processing section 47 performs various types of necessary image processing such as gradation processing on the B-mode image signal input from the DSC 46 and then sends out the B-mode image signal to the display controller 22 and the stool detection unit 24. Hereinafter, the B-mode image signal that has been subjected to image processing by the image processing section 47 is referred to as an ultrasound image.

The main body controller 26 controls each unit of the apparatus main body 2 and the ultrasound probe 1 in accordance with a program recorded in advance, or the like.

The input device 27 accepts an input operation from a user and sends out input information to the main body controller 26. The input device 27 is composed of, for example, a device that is used for the user to perform an input operation, such as a keyboard, a mouse, a trackball, a touchpad, or a touch panel.

The stool detection unit 24 analyzes the ultrasound image generated by the image generation unit 21 to perform processing of detecting the stool inside the subject shown in the ultrasound image. The stool detection unit 24 stores, for example, a plurality of template images related to the stool inside the subject, and can detect the stool through a so-called template matching method of searching the ultrasound image using the plurality of template images. The stool detection unit 24 can also detect the stool from the ultrasound image using, for example, a so-called machine learning model that has been trained using a large number of ultrasound images showing the stool inside the subject.

The selection button generation unit 25 generates a selection button that is displayed on the monitor 23 and that is a user interface which is used for the user to select via the input device 27 whether or not to operate the stool detection unit 24 and whether or not to display the detection result of the stool by the stool detection unit 24 on the monitor 23.

The display controller 22 performs predetermined processing on the ultrasound image or the like generated by the image generation unit 21 and displays the ultrasound image or the like on the monitor 23, under the control of the main body controller 26.

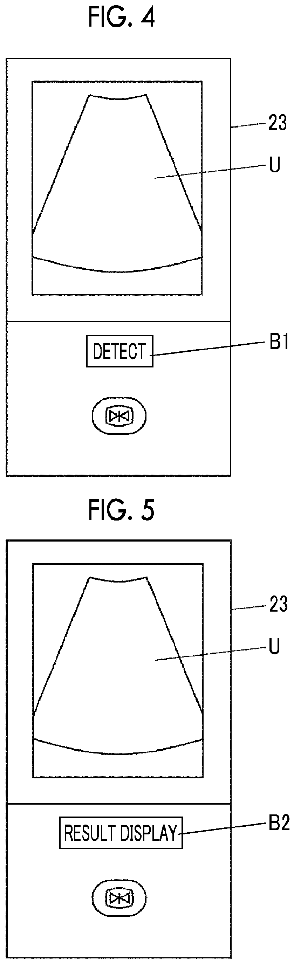

In addition, the display controller 22 displays the selection button generated by the selection button generation unit 25 on the monitor 23 under the control of the main body controller 26. For example, as shown in Fig. 4, the display controller 22 can display a selection button B1 for operating the stool detection unit 24 on the monitor 23 together with the ultrasound image U generated by the image generation unit 21. In the example of Fig. 4, the selection button B1 with the text "detect" displayed thereon is disposed on the monitor 23. In a case where the user selects the selection button B1 via the input device 27, the stool detection unit 24 can start the processing of detecting the stool from the ultrasound image U.

In addition, the display controller 22 displays the selection button on the monitor 23 in a display mode corresponding to the detection result by the stool detection unit 24 while the stool detection unit 24 is performing the processing of detecting the stool.

In a case where the stool is not detected by the stool detection unit 24, the display controller 22 can display, for example, as shown in Fig. 5, a selection button B2 having the text "result display" on the monitor 23. In a case where the user selects the selection button B2 via the input device 27, the display controller 22 can display, for example, a message indicating that no stool is detected on the monitor 23. In this case, the display controller 22 can delete the message indicating that no stool is detected from the monitor 23 in a case where the selection button B2 is selected again by the user as a trigger. In addition, in a case where the stool is not detected by the stool detection unit 24, the display controller 22 also cannot perform a particular display of the matter related to the detection result on the monitor 23 even in a case where the selection button B1 is selected.

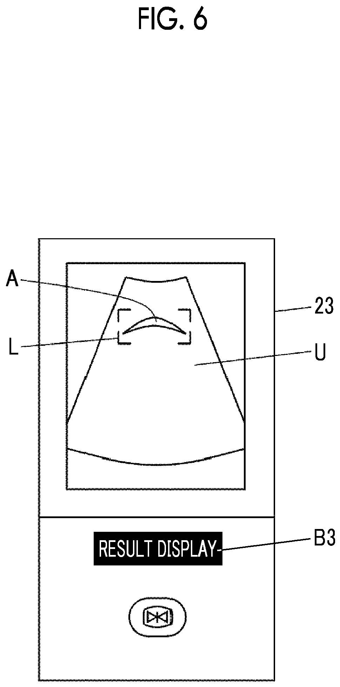

Further, in a case where the stool is detected by the stool detection unit 24, the display controller 22 can display, for example, as shown in Fig. 6, a selection button B3 on the monitor 23 in a display mode different from that of the selection button B2 in a case where the stool is not detected. The display mode different from that of the selection button B2 includes, for example, a color, a shape, a size, a frame line, a blinking state, and the like different from those of the selection button B2. In a case where the user selects the selection button B3 via the input device 27, the display controller 22 can display, for example, a presence range of a stool A within the ultrasound image U on the monitor 23 with a surrounding frame line L1, as the detection result of the stool detection unit 24. In this case, the display controller 22 can delete the frame line L1 from the monitor 23 in a case where the selection button B3 is selected again by the user as a trigger. The frame line L1 can be composed of, for example, a plurality of bending lines as shown in Fig. 6 surrounding the presence range of the stool A, or can also be composed of a closed line. Further, the shape of the frame line L1 is not particularly limited.

In this way, the user can easily grasp the detection result of the stool A by the stool detection unit 24 by confirming the display mode of the selection button B2 or B3, and can easily confirm the detection result of the stool A in detail by selecting the selection button B2 or B3.

The monitor 23 performs various kinds of display under the control of the display controller 22. The monitor 23 can include, for example, a display device such as a liquid crystal display (LCD) or an organic electroluminescence (EL) display.

Although the processor 32 including the image generation unit 21, the display controller 22, the stool detection unit 24, the selection button generation unit 25, and the main body controller 26 is composed of a central processing unit (CPU) and a control program for causing the CPU to perform various types of processing, the processor 32 may be composed of a field programmable gate array (FPGA), a digital signal processor (DSP), an application specific integrated circuit (ASIC), a graphics processing unit (GPU), or other integrated circuits (ICs), or may be composed of a combination thereof.

Further, the image generation unit 21, the display controller 22, the stool detection unit 24, the selection button generation unit 25, and the main body controller 26 of the processor 32 can also be configured by being integrated partially or entirely into one CPU or the like.

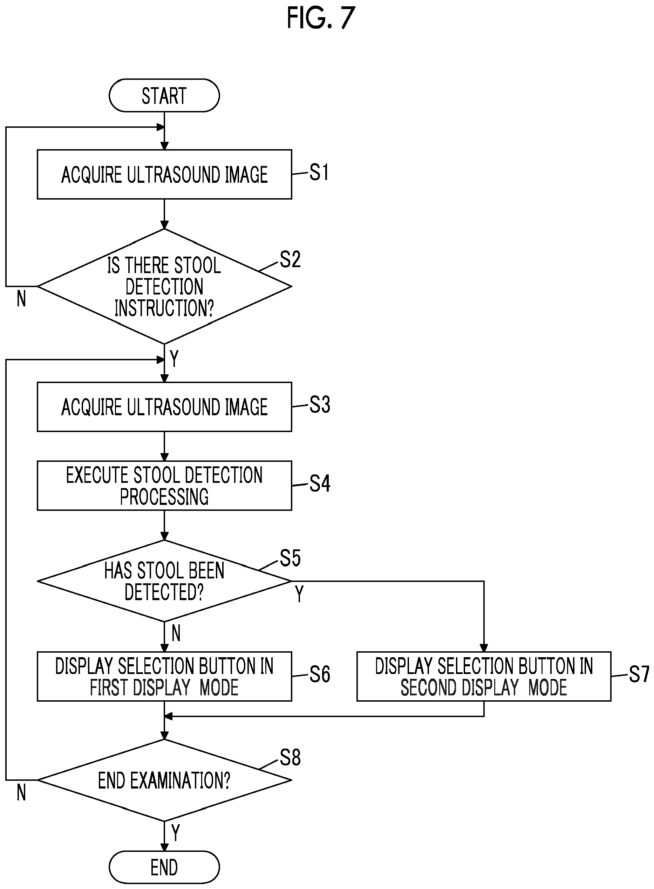

Next, an example of the operation of the ultrasound diagnostic apparatus according to the embodiment will be described using the flowchart of Fig. 7.

First, in step S1, in a state in which the user disposes the ultrasound probe 1 on the body surface of the subject, the image acquisition unit 31 acquires the ultrasound image U of the large intestine of the subject. In this case, the transducer array 11 of the ultrasound probe 1 transmits the ultrasound beam into the subject and receives the ultrasound echo from the inside of the subject, thereby generating the reception signal. The transmission and reception circuit 12 of the image acquisition unit 31 performs so-called reception focus processing on the reception signal to generate the sound ray signal, under the control of the main body controller 26. The sound ray signal generated by the transmission and reception circuit 12 is sent out to the image generation unit 21. The image generation unit 21 generates the ultrasound image U using the sound ray signal sent out from the transmission and reception circuit 12.

In this case, for example, as shown in Fig. 4, the display controller 22 displays the acquired ultrasound image U and the selection button B1 which is a user interface for operating the stool detection unit 24 on the monitor 23.

Next, the main body controller 26 determines whether or not there is an instruction to detect the stool A from the user. For example, in a case where the selection button B1 displayed on the monitor 23 is selected by the user via the input device 27, the main body controller 26 can determine that there is an instruction to detect the stool A from the user. In addition, for example, in a case where the selection button B1 displayed on the monitor 23 is not particularly selected by the user, the main body controller 26 can determine that there is no instruction to detect the stool A from the user.

In a case where it is determined in step S2 that there is no instruction to detect the stool A from the user, the process returns to step S1, and the ultrasound image U is newly acquired. In this manner, as long as it is determined in step S2 that there is no instruction to detect the stool A from the user, the processing of steps S1 and S2 is repeated.

In a case where it is determined in step S2 that there is an instruction to detect the stool A from the user, the process proceeds to step S3. In step S3, the image acquisition unit 31 acquires the ultrasound image U of the large intestine of the subject in the same manner as in step S1.

In subsequent step S4, the stool detection unit 24 executes processing of detecting the stool A from the ultrasound image U by analyzing the ultrasound image U acquired in step S3. In this case, the stool detection unit 24 can execute processing of detecting the stool A from the ultrasound image U through, for example, a template matching method or a method using a machine learning model.

In step S5, the main body controller 26 determines whether or not the stool A is detected in the stool detection processing in step S4. In a case where the stool A is detected in the stool detection processing in step S4, for example, information indicating that the stool A is detected is transmitted from the stool detection unit 24 to the main body controller 26, and the main body controller 26 can determine that the stool A is detected based on this information. In a case where the stool A is not detected in the stool detection processing in step S4, for example, information indicating that no stool A is detected is transmitted from the stool detection unit 24 to the main body controller 26, and the main body controller 26 can determine that no stool A is detected based on this information.

In a case where it is determined in step S5 that no stool A is detected, the process proceeds to step S6. In step S6, for example, as shown in Fig. 5, the display controller 22 displays on the monitor 23 the selection button B2 that is a user interface for displaying the detection result in step S4 on the monitor 23 in a first display mode, under the control of the main body controller 26. In a case where the user selects the selection button B2 via the input device 27, the display controller 22 can display, for example, a message indicating that no stool A is detected on the monitor 23. Further, for example, in a case where the user selects the selection button B2 again, the display controller 22 can delete the message from the monitor 23.

In a case where it is determined in step S5 that the stool A is detected, the process proceeds to step S7. In step S7, for example, as shown in Fig. 6, the display controller 22 displays on the monitor 23 the selection button B3 that is a user interface for displaying the detection result in step S4 on the monitor 23 in a second display mode different from the first display mode, under the control of the main body controller 26. In a case where the user selects the selection button B3 via the input device 27, the display controller 22 can display on the monitor 23, for example, the surrounding frame line L1 indicating the presence range of the stool A. Further, for example, in a case where the user selects the selection button B3 again, the display controller 22 can delete the frame line L1 from the monitor 23.

In this way, since the display controller 22 displays the selection buttons B2 and B3 on the monitor 23 in different display modes depending on whether or not the stool A is detected, the user can easily grasp the detection result of the stool A by confirming the display modes of the selection buttons B2 and B3.

In step S8 following steps S6 and S7, the main body controller 26 determines whether or not to end the examination. The main body controller 26 can determine to end the examination, for example, in a case where an instruction to end the examination is input from the user via the input device 27. In addition, the main body controller 26 can determine to continue the examination, for example, in a case where no particular instruction to end the examination is input from the user via the input device 27.

In a case where it is determined in step S8 to continue the examination, the process returns to step S3, and the ultrasound image U of the large intestine of the subject is newly acquired. In subsequent step S4, the processing of detecting the stool A is executed for the ultrasound image U acquired in step S3, and it is determined whether or not the stool A is detected in step S5. In a case where it is determined in step S5 that no stool A is detected, the selection button B2 is displayed on the monitor 23 in the first display mode in step S6. In a case where it is determined in step S5 that the stool A is detected, the selection button B3 is displayed on the monitor 23 in the second display mode in step S7. In this manner, the processing of steps S3 to S8 is repeated as long as it is determined in step S8 to continue the examination.

In a case where it is determined in step S8 to end the examination, the operation of the ultrasound diagnostic apparatus according to the flowchart of Fig. 7 is completed.

From the above, with the ultrasound diagnostic apparatus of Embodiment 1, the stool detection unit 24 executes the processing of detecting the stool A from the ultrasound image U, the selection button generation unit 25 generates the selection buttons B2 and B3 which are used for the user to select whether or not to display the detection result of the stool A by the stool detection unit 24, and the display controller 22 displays the selection buttons B2 and B3 on the monitor 23 in display modes corresponding to the detection result of the stool A by the stool detection unit 24. Therefore, the user can easily confirm the detection result without displaying the detailed detection result of the stool A on the screen of the monitor 23.

Although it has been described that the transmission and reception circuit 12 is provided in the ultrasound probe 1, the transmission and reception circuit 12 may be provided in the apparatus main body 2.

In addition, although it has been described that the image generation unit 21 is provided in the apparatus main body 2, the image generation unit 21 may be provided in the ultrasound probe 1.

Further, the apparatus main body 2 may be a so-called stationary type, a portable type that is easy to carry, or a so-called handheld type that is composed of, for example, a smartphone or a tablet type computer. As described above, the type of the device that constitutes the apparatus main body 2 is not particularly limited.

In addition, the main body controller 26 can perform control to stop the stool detection unit 24, for example, as a trigger, in a case where the user selects the selection button B2 or B3 through a predetermined method such as continuing to select the selection button B2 or B3 for a predetermined time or longer. As a result, the power of the ultrasound diagnostic apparatus can be saved in a situation in which the detection processing of the stool A is unnecessary.

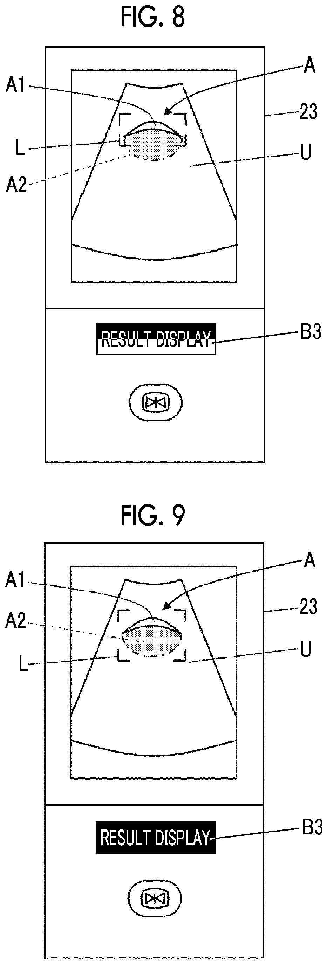

In addition, in general, in a case where the stool A is hard, ultrasound waves have difficulty in passing through the stool A. Therefore, as shown in Fig. 8, it is known that a portion where the stool A is present in the ultrasound image U has a high-brightness part A1 located at a shallow part of the stool A and a low-brightness region A2 is depicted on the deep side

The stool detection unit 24 can detect the presence or absence of the stool A in the deep-side region A2 of the high-brightness part A1 of the stool A on the ultrasound image U through, for example, a template matching method or a method using a machine learning model. In this case, as shown in Figs. 8 and 9, the display controller 22 can change the display mode of the selection button B3 according to the presence or absence of the stool A in the deep-side region A2 detected by the stool detection unit 24.

Figs. 8 and 9 show that the change range of the display mode of the selection button B3 changes according to the presence or absence of the stool A in the deep-side region A2 detected by the stool detection unit 24. In the example of Fig. 8, since the stool A is not detected in the deep-side region A2 of the high-brightness part A1, the upper half of the selection button B3 is colored and displayed on the monitor 23. In the example of Fig. 9, since the stool A is detected in the deep-side region A2 of the high-brightness part A1, the entire selection button B3 is colored and displayed on the monitor 23.

In this manner, by changing the display mode of the selection button B3 according to the presence range of the stool A, the user can easily grasp the detection result of the stool A in detail.

Further, although it has been described that the display controller 22 displays one selection button B1, B2, or B3 on the monitor 23, for example, the selection button B1 for selecting whether or not to operate the stool detection unit 24 and the selection button B2 or B3 for selecting whether or not to display the detection result by the stool detection unit 24 can also be displayed together on the monitor 23.

Further, it has been described that the message indicating that no stool A is detected is displayed on the monitor 23 in a case where the selection button B2 is selected when the stool A is not detected by the stool detection unit 24. However, in a case where the stool is detected by the stool detection unit 24 in this state, the display controller 22 can display the detected stool A with emphasis on the monitor 23 and can display the selection button B3 as shown in Fig. 6 on the monitor in a display mode different from that of the selection button B2 as shown in Fig. 5. As described above, in the present invention, the detection processing of the stool A by the stool detection unit 24 is performed in real time regardless of whether or not the selection button B2 or B3 is selected, the displays of the selection buttons B2 and B3 corresponding to the detection result can be switched in real time.

In addition, in a case where the selection button B2 is selected in a state in which the stool A is not detected by the stool detection unit 24, the display controller 22 can also indicate that no stool Ais detected by not displaying the frame line L1, instead of displaying the message indicating that no stool A is detected on the monitor 23.

### Embodiment 2

The ultrasound diagnostic apparatus of the embodiment of the present invention can also automatically determine the property of the detected stool A.

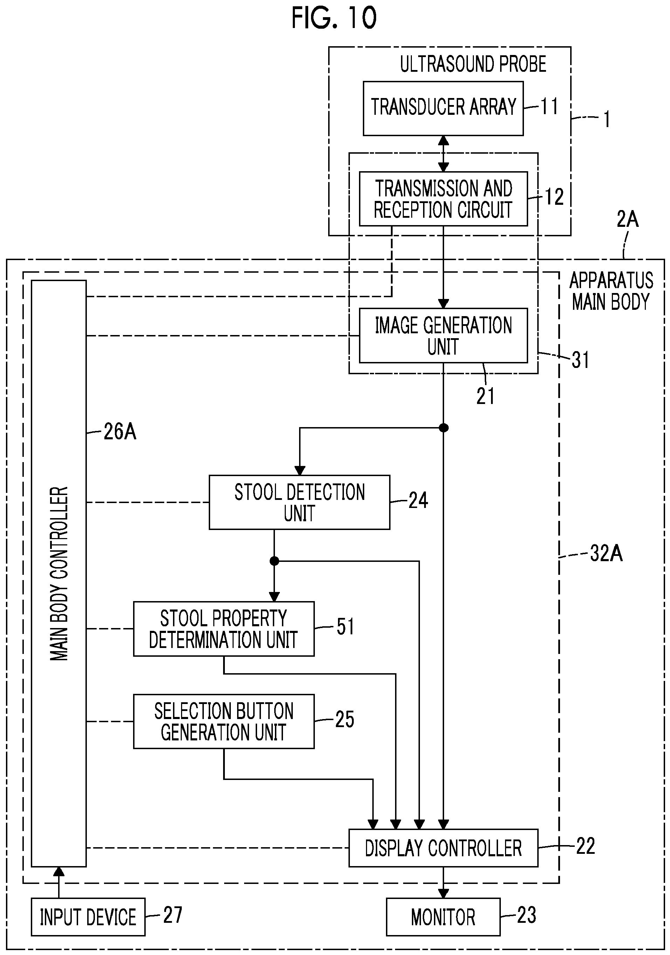

Fig. 10 shows a configuration of an ultrasound diagnostic apparatus of Embodiment 2. The ultrasound diagnostic apparatus of Embodiment 2 comprises an apparatus main body 2A instead of the apparatus main body 2 with respect to the ultrasound diagnostic apparatus of Embodiment 1 shown in Fig. 1. The apparatus main body 2A further comprises a stool property determination unit 51 and comprises a main body controller 26A instead of the main body controller 26, with respect to the apparatus main body 2 in Embodiment 1.

In the apparatus main body 2A, the stool property determination unit 51 is connected to the stool detection unit 24 and the main body controller 26A. The stool property determination unit 51 is connected to the display controller 22. In addition, the image generation unit 21, the display controller 22, the stool detection unit 24, the selection button generation unit 25, the main body controller 26A, and the stool property determination unit 51 constitute a processor 32A for the apparatus main body 2A.

The stool property determination unit 51 determines the property of the stool A detected by the stool detection unit 24. Here, it is generally known that a hard stool is shown as a so-called crescent-shaped high-brightness region in the ultrasound image U because ultrasound waves have difficulty in passing through the stool A and most of the ultrasound waves are reflected at the shallow part of the stool A, that a normal stool is shown as a so-called semicircular high-brightness region in the ultrasound image U because ultrasound waves pass through more easily than in the hard stool, and that a soft stool is shown as a so-called all-around low-brightness region in the ultrasound image U because ultrasound waves easily pass through the stool A. As described above, the appearance of the stool A in the ultrasound image U differs depending on the properties of the stool A. The stool property determination unit 51 can acquire the property of the stool A through, for example, a template matching method. The stool property determination unit 51 can also acquire the property of the stool A using, for example, a machine learning model that has learned a large amount of ultrasound images U showing the stool A and information regarding their properties of the stool A.

The stool property determination unit 51 can also determine the properties based on a so-called Bristol stool scale as the properties of the stool A. The Bristol stool scale classifies the properties of the stool A into seven states: "pellet-like stools" which are hard and pellet-like stools, like rabbit droppings; "hard stools" which are sausage-shaped but hard stools; "slightly hard stools" which are sausage-like stools with cracks on the surface; "normal stools" which are smooth and soft sausage-shaped or coiled stools, like a snake; "slightly soft stools" which are soft semi-solid stools with distinct wrinkles; "muddy stools" which are irregular small-piece stools or muddy stools with a loose boundary; and "watery stools" which are watery liquid-like stools with no solid pieces.

The display controller 22 stores predetermined display modes of the selection button B3 for a plurality of properties of the stool A, and can change the display mode of the selection button B3 according to the property of the stool A determined by the stool property determination unit 51. For example, the display controller 22 can display the selection button B3 in red color in a case where the stool A is determined to be a hard stool by the stool property determination unit 51, can display the selection button B3 in blue color in a case where the stool A is determined to be a normal stool by the stool property determination unit 51, and can display the selection button B3 in green color in a case where the stool A is determined to be a soft stool by the stool property determination unit 51.

From the above, with the ultrasound diagnostic apparatus of Embodiment 2, the stool property determination unit 51 automatically determines the property of the stool A detected by the stool detection unit 24, and the display controller 22 changes the display mode of the selection button B3 according to the property of the stool A determined by the stool property determination unit 51. Therefore, the user can easily grasp not only the presence or absence of the detection of the stool Abut also the property of the stool A shown in the ultrasound image U by confirming the display mode of the selection button B3.

### Explanation of References

1: ultrasound probe
2, 2A: apparatus main body
11: transducer array
12: transmission and reception circuit
21: image generation unit
22: display controller
23: monitor
24: stool detection unit
25: selection button generation unit
26, 26A: main body controller
27: input device
31: image acquisition unit
32, 32A: processor
41: pulsar
42: amplification section
43: AD conversion section
44: beam former
45: signal processing section
46: DSC
47: image processing section
A: stool
A1: high-brightness part
A2: region
B1 to B3: selection button
L: frame line
U: ultrasound image

## Claims

1. An ultrasound diagnostic apparatus comprising:
an ultrasound probe (1);
an image acquisition unit (31) configured to acquire an ultrasound image of a subject using the ultrasound probe (1);
a monitor (23) configured to display the ultrasound image;
a stool detection unit (24) configured to detect a stool from the ultrasound image;
a selection button (25) displayed on the monitor (25) and used for a user to select whether or not to display a detection result by the stool detection unit (24); and
a display controller (22) configured to display the selection button (25) on the monitor (23) in a display mode corresponding to the detection result by the stool detection unit (24).

2. The ultrasound diagnostic apparatus according to claim 1, further comprising:
a stool property determination unit (51) configured to determine a property of the stool detected by the stool detection unit (24),
wherein the display controller (22) is configured to change the display mode of the selection button (25) according to the property of the stool determined by the stool property determination unit (51).

3. The ultrasound diagnostic apparatus according to claim 1 or 2,
wherein the stool detection unit (24) is configured to detect presence or absence of the stool in a deep-side region of a high-brightness part on the ultrasound image, and
the display controller (22) is configured to change the display mode of the selection button (25) according to the presence or absence of the stool in the deep-side region detected by the stool detection unit (24).

4. The ultrasound diagnostic apparatus according to claim 3,
wherein the display controller (22) is configured to change a change range of the display mode on the selection button (25) according to a presence range of the stool in the deep-side region detected by the stool detection unit (24).

5. The ultrasound diagnostic apparatus according to any one of claims 1 to 4,
wherein the display controller (22) is configured to display a presence range of the stool detected by the stool detection unit (24) on the monitor (23) with a surrounding frame line.

6. The ultrasound diagnostic apparatus according to any one of claims 1 to 5,
wherein the display controller (22) is configured to display on the monitor (23) that no stool is detected in a case where the stool is not detected by the stool detection unit (24).

7. A control method of an ultrasound diagnostic apparatus including an ultrasound probe (1), an image acquisition unit (31), a monitor (23), a stool detection unit (24), a selection button (25), and a display controller (22), the control method comprising:
causing the image acquisition unit (31) to acquire an ultrasound image of a subject using the ultrasound probe (1);
causing the monitor (23) to display the ultrasound image;
causing the stool detection unit (24) to detect a stool from the ultrasound image; and
causing the display controller (22) to display the selection button (25) used for a user to select whether or not to display a detection result of stool detection, on the monitor (23) in a display mode corresponding to the detection result of the stool detection.
